(19) Europäisches Patentamt
European Patent Office
Office européen des brevets

(11) Numéro de publication: **0 121 828**
**B1**

(12) # FASCICULE DE BREVET EUROPEEN

(45) Date de publication du fascicule du brevet: **19.11.87**

(21) Numéro de dépôt: **84102912.7**

(22) Date de dépôt: **16.03.84**

(51) Int. Cl.⁴: **C 07 C 31/135**, C 07 C 29/17, C 07 C 29/136, A 61 K 7/46 // C07C45/62, C07C49/403, C07C29/42

(54) **Alcools aliphatiques, leur préparation et leur utilisation à titre d'ingrédients parfumants.**

(30) Priorité: **12.04.83 CH 1963/83**
**30.08.83 CH 4743/83**

(43) Date de publication de la demande: **17.10.84 Bulletin 84/42**

(45) Mention de la délivrance du brevet: **19.11.87 Bulletin 87/47**

(84) Etats contractants désignés: **CH DE FR GB LI NL**

(56) Documents cités:
**DE - A - 2 455 761**
**FR - A - 2 418 214**

(73) Titulaire: **FIRMENICH SA, 1, route des Jeunes, CH-1211 Genève 8 (CH)**

(72) Inventeur: **Schulte-Elte, Karl-Heinrich, Dr., 44, chemin de Carabot, CH-1213 Onex (CH)**
Inventeur: **Pamingle, Hervé, 20, avenue Henri-Dunant, CH-1205 Genève (CH)**

(74) Mandataire: **Salvadori, Giuseppe, Dr., c/o Firmenich S.A. Case Postale 239, CH-1211 Genève 8 (CH)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 121 828 B1

**Description**

La présente invention a trait au domaine de la parfumerie. Elle concerne plus particulièrement certains dérivés méthylés nouveaux du 10-éthyl-tétrahydroionol, lesquels composés sont décrits à l'aide de la formule générale suivante

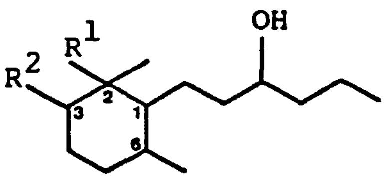

(I)

dans laquelle chacun des symboles $R^1$ et $R^2$ sert à désigner un radical méthyle ou dans laquelle $R^1$ désigne un radical éthyle et $R^2$ un radical méthyle ou l'hydrogène.

L'invention concerne en outre l'utilisation desdits composés en tant qu'ingrédients parfumants ainsi qu'un procédé pour leur préparation.

L'invention est définie comme indiqué aux revendications.

L'art antiérieur fait état des propriétés odorantes, en particulier fixatives, du 1-(2,6,6-triméthylcyclohexyl)-hexan-3-ol[voir DE-AS 2 807 584] également connu sous le nom de 10-éthyl-tétrahydroionol. L'art antérieur fait également état de certains dérivés dudit composé comportant soit une chaîne latérale hydroxylée ramifiée soit une chaîne hydroxylée «raccourcie» à cinq atomes de carbone [Dragoco Report 199 (1980)].

Les antériorités citées ci-dessus ne font aucune mention particulière de l'intérêt qui pourrait être représenté par l'un ou l'autre des composés de formule (I) et, qui plus est, tendent à montrer que les composés autres que le 10-éthyl-tétrahydroionol présentent des propriétés odorantes sensiblement moins développées que celles montrées par ce dernier.

C'est donc de manière surprenante que nous avons pu constater que non seulement les composés méthylés de formule (I) possédaient des caractères odorants fort intéressants, mais que leurs propriétés étaient en effet supérieures à celles montrées par le 10-éthyl-tétrahydroionol tant du point de vue de leur qualité que de leur puissance. Nous avons en outre découvert de façon inattendue qu'il existait une différence marquée entre les qualités olfactives des différents isomères de cette série de composés, les isomères cyclaniques trans étant jugés nettement supérieurs à leurs isomères cis correspondants.

Tout au long de cette description, lorsqu'il est fait mention d'isomérie cyclanique cis ou trans, on entend se référer à la configuration particulière du groupe hydroxyhexyle par rapport au groupe monométhyle relié au noyau hexanique.

Ainsi, l'un des aspects particuliers de la présente invention a trait à l'utilisation, en tant qu'ingrédients parfumants, des composés de formule (I) dans leur configuration trans ou des mélanges contenant des quantités prépondérantes (supérieures à 50% en poids) desdits composés accompagnés par des quantités inférieures (inférieures à 50% en poids) de leurs isomères cis correspondants.

D'autre part, nous avons également pu établir que dans le cas des composés (I) comportant un substituant $R^1$ = éthyle et/ou $R^2 = CH_3$, les isomères particuliers ayant en même temps une configuration équatoriale des différents substituants dans les positions 1, 2 et/ou 3 et 6 du noyau hexanique possédaient des caractères odorants plus marqués vis-à-vis des composés correspondants présentant une autre configuration.

Ainsi, par exemple, le composé de structure

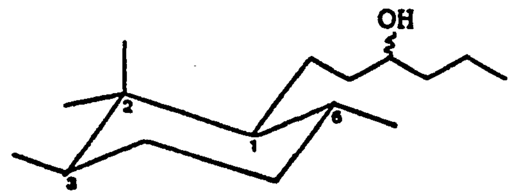

dont les substituants, dans les positions 1,3 et 6 du cycle hexanique, occupent la position équatoriale possèdent une puissante odeur à caractère stéroïdale, animal et boisé, voire ambré. Ce caractère, accompagné d'une note de transpiration, se retrouve également dans le composé de structure

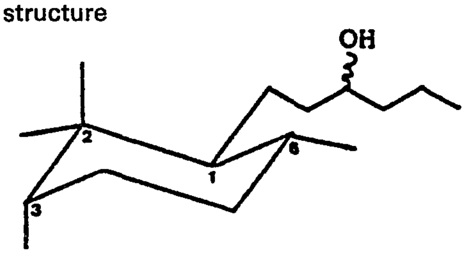

ayant le groupe méthyle en position axiale. Sa puissance olfactive est cependant moins prononcée que celle du composé précédent.

Grâce à la présente invention, il est désormais possible de préparer un produit essentiellement constitué par les isomères olfactivement les plus intéressants.

Le procédé suivant l'invention consiste en la réduction d'un carbinol allénique de formule

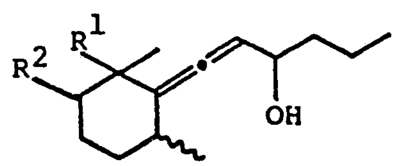

(II)

dans laquelle ~ indique une liaison simple carbone-carbone d'isomérie cis ou trans, au moyen d'une hydrogénation catalytique, en phase homogène ou hétérogène, à une pression supérieure à la pression atmosphérique et en présence d'un catalyseur constitué par un métal noble.

A titre de catalyseur on peut mentionner le palladium, le platine, éventuellement sur charbon actif, ou le rhodium.

La pression employée peut varier dans une gamme de valeurs assez étendue et être comprise, par exemple, entre 25 et 150 atmosphères.

Le produit que l'on obtient ainsi est constitué pour l'essentiel de l'isomère trans désiré de formule

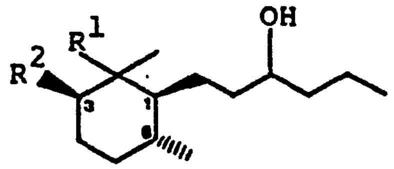

(a)

accompagné de quantités mineures des isomères de formule

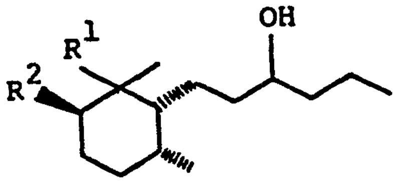

(b)

et de formule

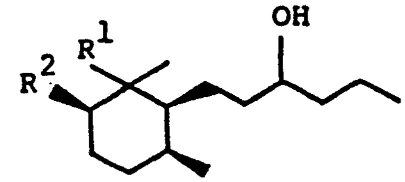

(c)

et

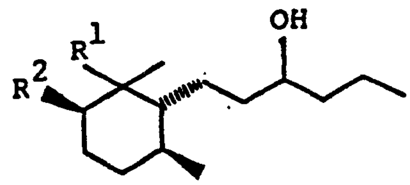

(d)

Le mélange obtenu peut être enrichi davantage en l'isomère (a) désiré au moyen, par exemple, de séparation gaz-chromatographique.

Le carbinol allénique (II) utilisé comme produit de départ dans le procédé décrit plus haut peut être préparé par la méthode illustrée à l'aide du schéma que voici:

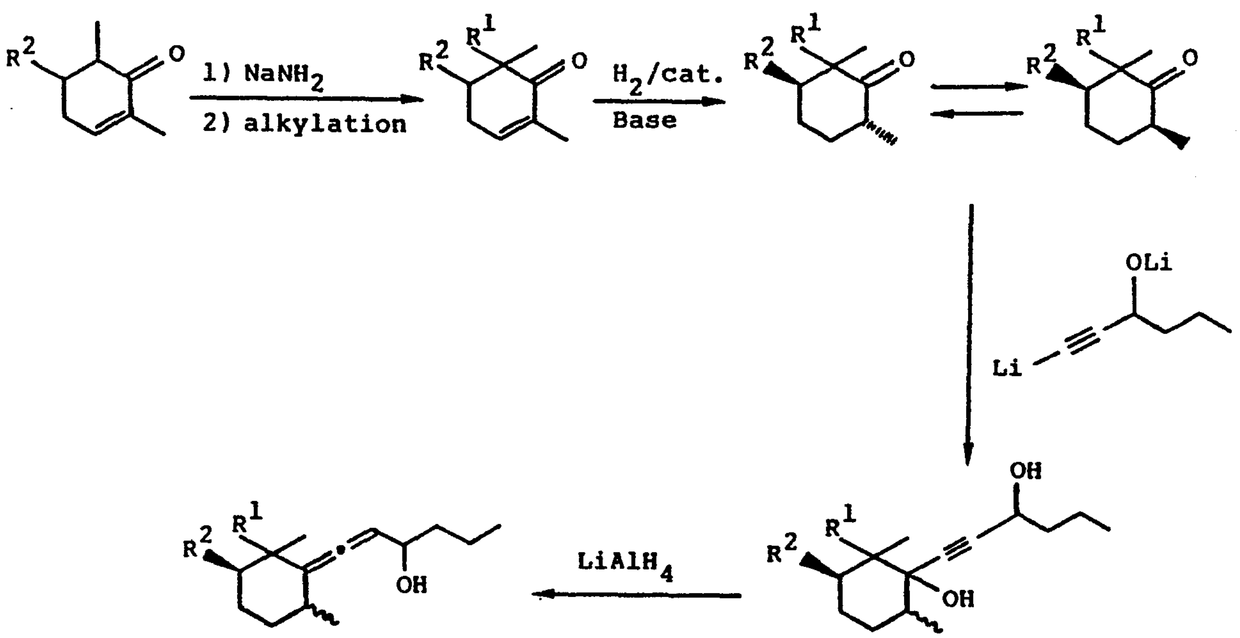


Pour toute utilisation pratique le mélange isomérique tel qu'obtenu directement du procédé décrit convient parfaitement pour l'étape suivante de réaction.

Comme indiqué plus haut, le procédé décrit sert à obtenir les composés (I) éminemment à structure tri-equatoriale vis-à-vis des substituants aux centres asymétriques 1, 3 et 6.

Un procédé original a été également développé pour la préparation des autres isomères à structure trans intéressant la parfumerie, lequel procédé est caractérisé par la réduction d'une cétone de formule

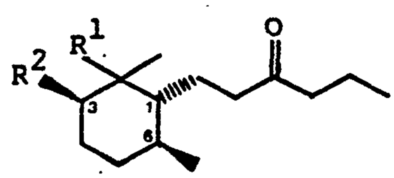

(III)

à l'aide d'un alumino-hydrure de métal alcalin pour fournir le carbinol désiré de formule

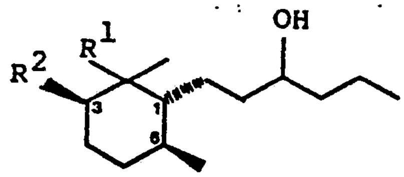

La cétone (III) utilisée comme produit de départ dans ce procédé peut être préparée ainsi:

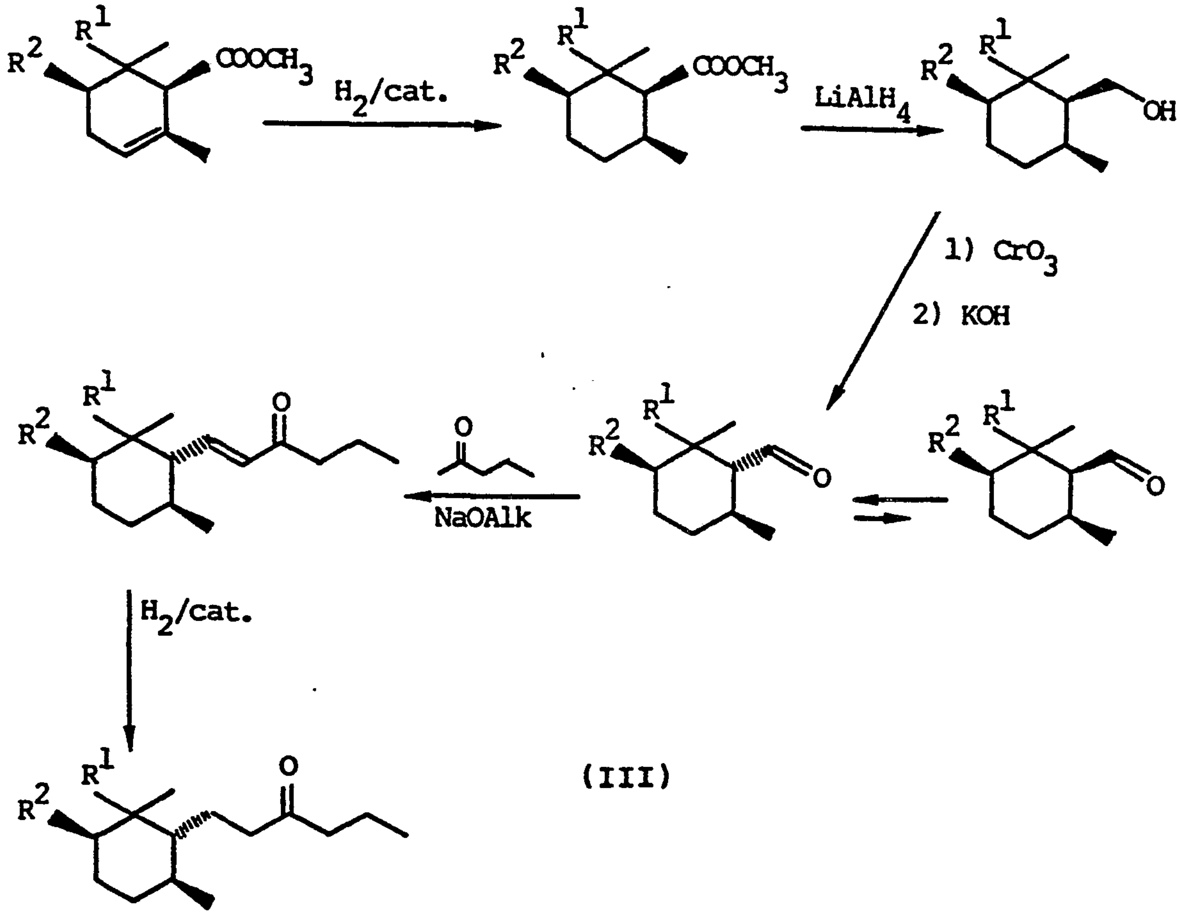


(III)

Les procédés mentionnés ci-dessus seront décrits en détail dans les exemples qui suivent.

Comme c'est souvent le cas dans des applications de parfumerie, les composés de formule (I) de l'invention peuvent être utilisés à des concentrations pouvant varier dans une gamme assez étendue de valeurs. Des concentrations de l'ordre de 1 à 10% en poids, par rapport au poids de la composition dans laquelle ils ont été incorporés, sont employées de préférence. De telles valeurs peuvent être inférieures à celles indiquées, notamment lors du parfumage d'articles divers tels les savons, les articles de toilette, les cosmétiques ou les détergents et les adoucisseurs textiles.

De par leurs propriétés olfactives particulières, les composés (I) peuvent trouver un domaine d'application fort étendu, soit par addition directe aux produits que l'on désire parfumer soit, plus fréquemment, par dilution préalable dans un solvant usuel, comme l'éthanol, l'anozol ou le phtalate diéthylique et en mélange avec d'autres ingrédients parfumants naturels ou synthétiques de nature variée.

Vis-à-vis du composé analogue connu ou 1-(2,6,6-triméthylcyclohexyl)-hexan-3-ol[mélange constitué pour l'essentiel par l'isomère cyclanique cis, du nom commercial de TIMBEROL (origine: Dragoco, Holzminden, RFA)], les composés de l'invention, notamment les composés ayant les substituants dans les positions 1, 2 et /ou 3 et 6 du cycle hexanique en position équatoriale, présentent une note plus franche et un caractère ambré et animal plus marqué. Leur puissance est également supérieure à cell du TIMBEROL, étant estimée à environ 20-25 fois celle de ce dernier composé.

L'invention est illustrée de manière plus détaillée par les exemples suivants (température en degrés centigrades).

Exemple 1

Préparation de 1-(2,2,3,6-tétraméthyl-1-cyclohexyl)-3-hexanol

2 G (8,5 mM) de 1-(2,2,3,6-tétraméthyl-1-cyclohexylidène)-1-hexène-3-ol dans 20 ml d'acétate d'éthyle ont été hydrogénés, en présence de 0,2 g de palladium à 5% sur charbon actif, dans un autoclave sous une pression d'environ 100 atm. d'hydrogène. Après filtration, le mélange a été concentré et distillé dans un four à boules (temp. du four : 130° environ) sous pression réduite.

On a ainsi obtenu 1,9 g (rend. 95%) d'un mélange contenant à raison d'environ 80% en poids de 1-(2,2,c-3,t-6-tétraméthyl-r-1-cyclohexyl)-3-hexanol et 10% de 1-(2,2,t-3,c-6-tétraméthyl-r-1-cyclohexyl)-3-hexanol accompagnés de quantités mineures de 1-(2,2,c-3,c-6-tétraméthyl-r-1cyclohexyl)-3-hexanol et de 1-(2,2-t-3,t-6-tétraméthyl-r-1-cyclohexyl)-3-hexanol.

Les caractères analytiques du 1-(2,2,c-3,t-6-tétraméthyl-r-1-cyclohexyl)-3-hexanol étaient les suivants:

IR: 3350 $cm^{-1}$;

RMN: 0,45 (m,1H); 0,65 (s,3H); 0,8–0,95 (m,12H); 1,0–1,65 (m,14H); 3,55 (m,2H) δ ppm;

SM: $M^+$=240; m/e: 222(3), 207(4), 197(4), 179(6), 161(1), 152(18), 145(1), 137(20), 131(1), 123(40), 103(46), 95(63), 83(93), 69(91), 55(100), 41(50).

1-(2,2,t-3,c-6-tétraméthyl-r-1-cyclohexyl)-3-hexanol

IR: 3350 $cm^{-1}$;

RMN (360 MHz; $CDCl_3$): 0,7 et 0,75 (3H, 2s); 0,82–0,95 (12H); 1,1–1,65 (14H, plusieurs m); 1,9 (1H, m); 3,6 (1H, m) δ ppm;

SM: $M^+$ = 240; m/e: 222(7), 207(16), 197(4), 179(8), 166(5), 152(18), 137(28), 123(35), 109(39), 95(46), 83(57), 69(66), 55(100), 42(71).

1-(2,2,c-3,c-6-tétraméthyl-r-1-cyclohexyl)-3-hexanol

IR: 3350 $cm^{-1}$;

RMN (360 MHz; $CDCl_3$): 0,7 et 0,72 (3H,2s); 0,82–0,9 (9H); 0,95 (3H,t); 1,1–1,7 (14H, plusieurs m); 1,9 (1H, m); 3,6 (1H, m) δ ppm;

SM: $M^+$ = 240; m/e: 222(9), 207(42), 197(2), 179(8), 166(10), 152(11), 137(50), 123(41), 109(41), 95(51), 83(48), 69(65), 55(100), 41(69).

Le 1-(2,2,3,6-tétraméthyl-1-cyclohexylidène)-1-hexène-3-ol, produit de départ dans le procédé décrit ci-dessus, peut être préparé ainsi.

a. 2,5,6,6-Tétraméthyl-2-cyclohexène-1-one

Ce composé a été préparé selon la méthode décrite dans DE-OS 2 444 585 avec un rendement de 95%.

b. 2,2,3,6-Tétraméthyl-1-cyclohexanone

152 G (1 M) de la cétone obtenue sous lettre a. ci-dessus ont été hydrogénés dans 500 ml de méthanol et en présence de 5 g de palladium à 5% sur charbon. Après absorption d'environ 23 lt d'hydrogène, le mélange a été filtré, concentré sous vide et distillé à l'aide d'une colonne Vigreux pour donner 123 g de la cétone désirée ayant Eb. 73-6°/5,32 × $10^2$ Pa; rend. 80%.

Le produit ainsi obtenu a été ensuite placé dans un réacteur sous azote et mis à reflux avec 100,8 g (0,9 M) de ter-butylate de potassium et 460 ml d'éthanol absolu.

Le mélange a été versé sur de la glace et extrait à l'éther, et les extraits organiques combinés ont été lavés avec de la saumure jusqu'à neutralité, séchés sur $Na_2SO_4$, filtrés, concentrés et distillés.

On a ainsi obtenu 111 g d'un mélange ayant Eb. 62-4°/5,32 × $10^2$ Pa (rend. 88%) constitué par 80% en poids de trans-2,2,3,6-tétraméthyl-1-cyclohexanone et 20% de l'isomère cis correspondant.

IR: 1710 $cm^{-1}$;

RMN (60 MHz): 0,8–1,15 (12H, 4s); 1,55–1,80 (4H, s); 1,8–2,2 (3H, m); 2,4–2,9 (6H, m) δ ppm;

SM: $M^+$ = 154(35); m/e: 136(1), 121(3), 112(17), 96(100), 84(46), 65(75), 55(35), 41(45), 27(50).

c. 1-(3-Hydroxy-1-hexynyle)-2,2,3,6-tétraméthyl-1-cyclohexanol

495 Ml (1,16 M) d'une solution de butyl-lithium à 15% dans l'hexane ont été ajoutés en 2 heures sous azote et sous vigoureuse agitation à un mélange, maintenu à −70°, de 51 g (0,52 M) d'hexyn-3-ol dans 250 ml de tétrahydrofuranne THF) anhydre.

Après addition, le mélange a été porté à température ambiante et laissé sous agitation pendant une nuit.

Une fois refroidi de nouveau à −70°, on y a introduit goutte à goutte 0,3 M du mélange obtenu sous lettre b. ci-dessus en solution dans 115 ml de THF et le tout a été maintenu sous agitation pendant 3 h à −70°, puis une nuit à température ambiante.

Le mélange réactionnel a été versé sur de la glace, extrait à l'éther, lavé jusqu'à neutralité avec de la saumure, séché sur $Na_2SO_4$, filtré, concentré et distillé. Le produit désiré a été ainsi obtenu avec un rendement de 65%.

IR: 3400 $cm^{-1}$;

RMN (60 MHz): 0,75–1,25 (15H, m); 1,25–2,15 (10H, m); 4,45 (1H, t) δ ppm;

SM: $M^+$ = 252; m/e: 234(1), 219(12), 210(8), 191(46), 177(9), 163(16), 149(66), 135(35), 121(31), 111(38), 96(67), 83(36), 69(60), 55(91), 43(25), 41(92).

d. 1-(2,3,6,6-Tétraméthyl-1-cyclohexylidène)-1-hexène-3-ol

0,22 M du diol obtenu sous lettre c. ci-dessus en solution dans 100 ml de THF ont été ajoutées goutte à goutte à une suspension maintenue sous azote de 9,3 g (0,232 M) de $LiAlH_4$ dans 400 ml de THF.

Le mélange réactionnel a été maintenu à reflux pendant 4 h, puis il a été agité une nuit à température ambiante, ensuite il a été hydrolysé avec précaution par addition successive de 9,3 ml d'eau, 9,3 ml d'une solution à 15% de NaOH et 27,9 ml d'eau.

Après filtration, le filtrat clair a été concentré

sous vide pour fournir un résidu qui, par distillation, a fourni le produit désiré ayant Eb. 63–70°/13,3 Pa.

IR: 3350 et 1960 $cm^{-1}$;

RMN (60 MHz): 0,73–1,10 (15H, m); 1,15–2,5 (10H, m); 3,9–4,3 (1H, m); 5,35 (1H, 2xd) δ ppm:

SM: $M^+$ = 236; m/e: 221(4), 203(5), 194(39), 175(18), 164(8), 149(54), 135(35), 121(84), 107(75), 95(55), 81(39), 71(65), 55(95), 43(100), 41(88).

Exemple 2

Préparation de 1-(2-éthyl-2,6-diméthyl-1-cyclohexyl)-3-hexanol

15 G (63,6 mM) de 1-(2-éthyl-2,6-diméthyl-1-cyclohexylidène)-1-hexène-3-ol dans 150 ml d'acide acétique ont été hydrogénés à température ambiante en présence de 100 mg de $PtO_2$. Après 6 h, on a observé une adsorption d'environ 2,9 lt d'hydrogène. Après filtration, le filtrat clair a été concentré, repris à l'éther et lavé successivement avec de l'eau, une solution à 10% de NaOH (2x), puis avec de la saumure jusqu'à neutralité. Une fois séché, filtré et concentré, le filtrat a été distillé pour fournir 13,1 g d'une fraction ayant Eb. 77–93°/13,3 Pa.

Un fractionnement à l'aide d'une colonne Vigreux a permis d'isoler une fraction du produit désiré constitué pour l'essentiel des isomères suivants:

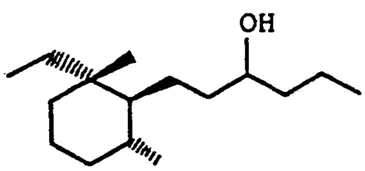
66%

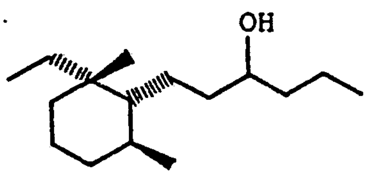
22%

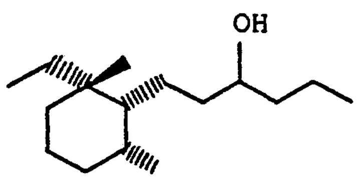
9%

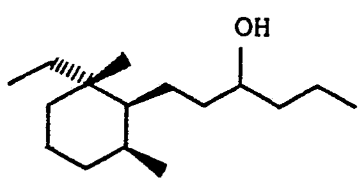
3%

Les caractéristiques analytiques du mélange isomérique obtenu étaient les suivantes:

IR: 3350 $cm^{-1}$;

RMN (360 MHz; $CDCl_3$): 0,59–0,65 (1H, m); 0,73–0,97 (12H, m); 1,0–1,65 (17H, m); 1,82–1,96 (1H, m); 3,5–3,61 (1H, m) δ ppm;

SM: $M^+$: 240; m/e: 222(10), 221(20), 193(61), 179(1), 165(3), 152(22), 137(26), 123(97), 109(70), 95(68), 83(63), 69(61), 55(100), 41(57).

Le 1-(2-éthyl-2,6-diméthyl-1-cyclohexylidène)-1-hexène-3-ol, produit de départ dans le procédé décrit ci-dessus, peut être préparé ainsi:

a. 2-Ethyl-1-(3-hydroxy-1-hexynyl)-2,6-diméthyl-1-cyclohexanol

40,2 G (0,41 M) de 1-hexyn-3-ol dans 200 ml de tétrahydrofuranne anhydre (THF) ont été introduits dans un réacteur tricol et refroidis à −70°. A cette température on y a ajouté sous agitation 390 ml (0,91 M) d'une solution de butyllithium à 15% dans l'hexane et la température a été ensuite amenée à environ 20° au cours d'une nuit. Une fois le mélange refroidi à nouveau à −70°, l'on y a ajouté goutte à goutte 40 g (0,26 M) d'une solution de 2-éthyl-2,6-diméthyl-cyclohexanone – mélange isomérique 75/25 des composés suivants:

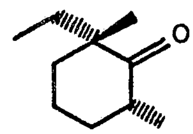
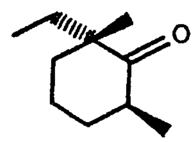

dans 90 ml de THF et le tout a été agité 3 h à −70° et une nuit à température ambiante.

Le mélange réactionnel a été versé sur de la glace, extrait à l'éther, lavé jusqu'à neutralité avec de la saumure, séché sur $Na_2SO_4$, filtré et concentré. Par distillation fractionnée, on a obtenu 27,3 g d'une fraction du produit désiré ayant Eb. 97–127°/13,3 Pa.

IR: 3400 $cm^{-1}$;

RMN (60 MHz; $CDCl_3$): 0,65–1,18 (12H); 1,2–1,85 (13H, m); 4,46 (1H, t, J=6) δ ppm;

SM: $M^+$ = 252; m/e: 201(0), 184(5), 155(25), 141(33), 127(73), 109(19), 96(26), 85(97), 69(37), 55(100), 41(69).

b. 1-(2-Ethyl-2,6-diméthyl-1-cyclohexylidène)-1-hexène-3-ol

27 G (0,107 M) du diol obtenu sous lettre a, ci-dessus dans 70 ml de THF anhydre ont été ajoutés goutte à goutte, sous agitation et en atmosphère d'azote, à une suspension de 4,28 g (0,107 M) de $LiA1H_4$ dans 200 ml de THF. Le mélange a été maintenu à reflux pendant 4 h, puis à température ambiante pendant une nuit. Avec précaution, on y a introduit ensuite successivement 4,28 ml d'eau, 4,28 ml d'une solution aqueuse à 15% de NaOH et enfin 12,84 ml d'eau. Après avoir été maintenu 1 h supplémentaire sous agitation, le mélange a été filtré, concentré et soumis à distillation fractionnée pour fournir 16,9 g du produit désiré ayant Eb. 62–69°/13,3 Pa. Ce produit était constitué par un mélange isomérique consistant en environ 2,5:1 des allènes de structure

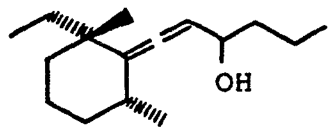

et

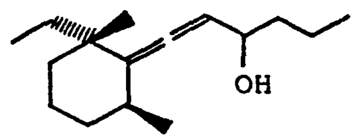

IR: 3375 et 1960 $cm^{-1}$;
RMN (60 MHz, $CDCl_3$): 0,60–1,15 (12H); 1,2–2,1 (13H, m); 3,95–4,3 (1H, m); 5,2–5,45 (1H, m) δ ppm;
SM: $M^+$ = 236; m/e: 219(1), 207(29), 194(14), 175(9), 165(11), 149(16), 135(100), 121(18), 107(39), 93(46), 81(33), 71(34), 55(73), 43(64), 41(51).

Exemple 3

Une composition parfumante de base a été préparée en mélangeant les ingrédients suivants (parties en poids):

| | |
|---|---|
| Iralia®1) | 120 |
| Cyclosia®base1) | 100 |
| Exaltolide®1) 10%* | 100 |
| Musc cétone | 80 |
| Musc xylène | 70 |
| Musc ambrette | 60 |
| Coumarine | 60 |
| Trichlorométhylphénylcarbinyle acétate | 50 |
| Essence de patchouli | 50 |
| Eugénol | 45 |
| Galbanum résinoïde | 35 |
| Ess. rose blanche synth. | 30 |
| Lilial® (L. Givaudan) | 30 |
| Acétate de vétyvéryle | 30 |
| Méthyl-eugénol | 20 |
| cis-10,10-Diméthyltricyclo [7.1.1.$0^{2,7}$]undéc-2-ène-4-one1)2) | 20 |
| β-Ionone | 10 |
| Essence de jasmin synth. | 10 |
| Résine de Bejoin Siam | 10 |
| Essence d'orange déterpénée | 10 |
| | 940 |

*dans le glycol dipropylique
1) origine: Firmenich SA, Genève
2) voir brevet CH 626 532

A l'aide de la base ci-dessus, on a préparé trois nouvelles compositions ainsi:

| | A | B | C |
|---|---|---|---|
| Composition de base | 94 | 94 | 94 |
| TIMBEROL1) | – | 6 | – |
| Produit de l'Exemple 1* | – | – | 6 |
| Anozol | 6 | – | – |
| | 100 | 100 | 100 |

*en solution à 5% dans l'anozol
1) origine: Dragoco, Holzminden, RFA

Les compositions obtenues ont été soumises pour évaluation olfactive à un groupe d'experts qui ont exprimé leur avis en choisissant la composition C comme étant la meilleure, celle dans laquelle la note était la plus chaude plus profonde et plus boisée. Sa puissance olfactive était supérieure à celle montrée par la composition de référence A et par la composition B obtenue à l'aide du produit connu de l'art antérieur.

Exemple 4

Deux bases détergentes en poudre ont été préparées en mélangeant les ingrédients suivants (parties en poids):

| | Composition | Composition avec perborate de sodium |
|---|---|---|
| Alkyl-benzènesulphonate de sodium linéaire (longeur de la chaîne $C_{11-5}$) | 8,0 | 6,4 |
| Alcool de suif éthoxylé (14EO) | 2,9 | 2,3 |
| Savon sodique (longueur de chaîne $C_{12-16}$ 13–26%; $C_{18-22}$ 74–87%) | 3,5 | 2,8 |
| Triphosphate de sodium | 43,8 | 35,0 |
| Silicate de sodium | 7,5 | 6,0 |
| Silicate de magnésium | 1,9 | 1,5 |
| Carboxyméthylcellulose | 1,2 | 1,0 |
| Sodium EDTA | 0,2 | 0,2 |
| Sulphate de sodium | 21,2 | 17,0 |
| Eau | 9,8 | 7,8 |
| Perborate de sodium | – | 20,0 |
| | 100,0 | 100,0 |

Lorsqu'on additionne à un échantillon de l'une des poudres détergentes ci-dessus le produit de l'Exemple 1, à raison d'environ 1% en poids, on lui confère une odeur boisée puissante et élégante.

Exemple 5

A l'aide du produit obtenu à l'Exemple 1, on a procédé au parfumage des articles suivants à la concentration indiquée:

| | |
|---|---|
| Eau de toilette | 5 % |
| Crème de jour | 0,4% |
| Crème de nuit | 0,4% |
| Shampoing | 0,5% |
| Déodorant aérosol | 1,2% |
| Laque pour cheveux | 0,3% |
| Savon[1)] | 0,5% |
| Talc | 0,5% |
| Poudre à récurer chlorée | 0,2% |

L'adjonction du produit en question servait à conférer à ces différents articles une note odorante boisée de très bonne stabilité et puisssance. Des essais de stabilité ont été effectués sur des échantillons de ces mêmes articles en les soumettant à un traitement à 40°C pendant 1 mois. Tous les échantillons examinés se sont révélés parfaitement stables.

**Revendications**

1. Composé de formule

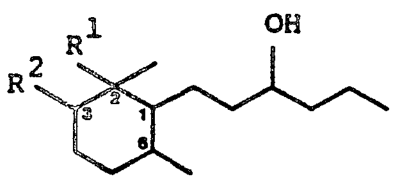

(I)

dans laquelle chacun des symboles $R^1$ et $R^2$ sert à désigner un radical méthyle ou dans laquelle $R^1$ désigne un radical éthyle et $R^2$ un radical méthyle ou l'hydrogène.

2. 1-(2,2,3,6-Tétraméthyl-1-cyclohexyl)-3-hexanol.

3. 1-(2-Ethyl-2,6-diméthyl-1-cyclohexyl)-3-hexanol.

4. 1-(2-Ethyl-2,3,6-triméthyl-1-cyclohexyl)-3-hexanol.

5. Composé selon la revendication 2 de formule

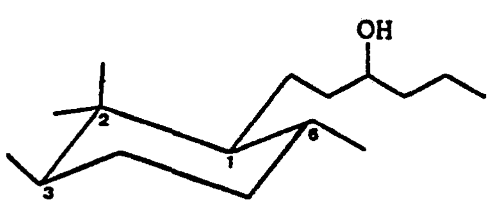

6. Composé selon la revendication 2 de formule

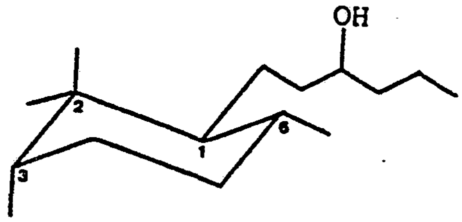

7. Utilisation d'un composé de formule (I) selon la revendication 1 à titre d'ingrédient parfumant.

8. Utilisation selon la revendication 7, caractérisée en ce que le composé de formule (I) se présente essentiellement sous sa forme isomérique trans définie par les formules suivantes:

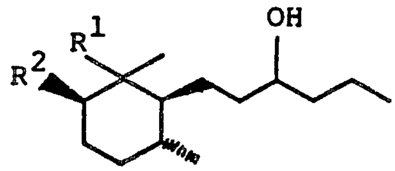

(a)

et

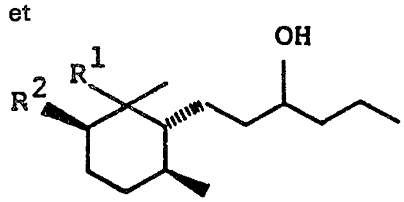

(d)

9. Procédé pour la préparation d'un composé de formule (I) selon la revendication 1, caractérisé en ce qu'on réduit un carbinol allénique de formule

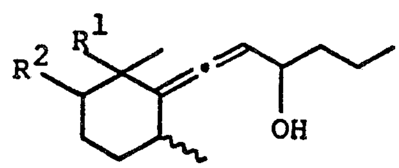

(II)

dans laquelle ~ indique une liaison simple carbone-carbone d'isomérie cis ou trans, au moyen d'une hydrogénation catalytique, en phase homogène ou hétérogène, à une pression supérieure à la pression atmosphérique et en présence d'un catalyseur constitué par un métal noble.

10. Procédé selon la revendication 9, caractérisé en ce que le catalyseur est constitué par du palladium ou du platine sur charbon.

11. Procédé pour la préparation d'un composé de formule (I) selon la revendication 1 essentiellement sous sa forme isomérique trans de formule

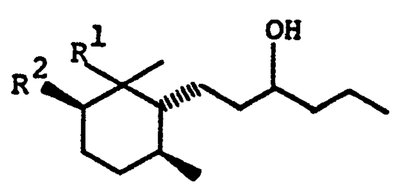

caractérisé en ce qu'on réduit une cétone de formule

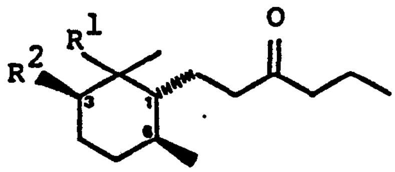

(III)

à l'aide d'un alumino-hydrure de métal alcalin.

**Patentansprüche**

1. Verbindung der Formel

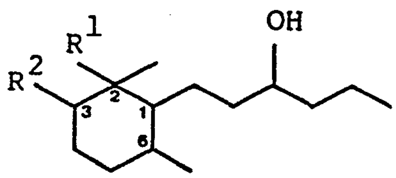

(I)

in welcher jedes der Symbole $R^1$ und $R^2$ ein Methylradikal darstellt oder in welcher $R^1$ ein Äthylradikal und $R^2$ ein Methylradikal oder ein Wasserstoff bezeichnet.

2. 1-(2,2,3,6-Tetramethyl-1-cyclohexyl)-3-hexanol.

3. 1-(2-Äthyl-2,6-dimethyl-1-cyclohexyl)-3-hexanol.

4. 1-(2-Äthyl-2,3,6-trimethyl-1-cyclohexyl)-3-hexanol.

5. Verbindung gemäss Anspruch 2 der Formel

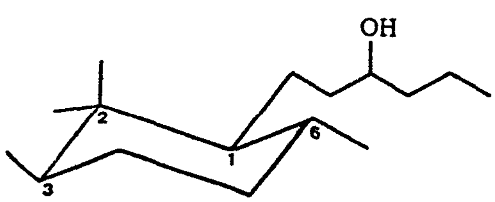

6. Verbindung gemäss Anspruch 2 der Formel

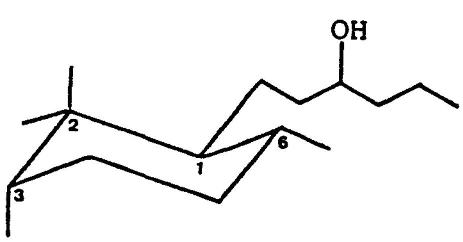

7. Verwendung einer Verbindung der Formel (I) gemäss Anspruch 1 als Parfümbestandteil.

8. Verwendung gemäss Anspruch 7, dadurch gekennzeichnet, dass die Verbindung der Formel (I) hauptsächlich als Trans-Isomer der Formel

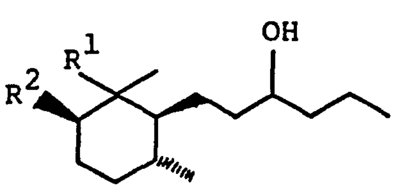

(a)

oder der Formel

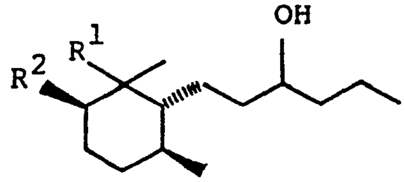

(d)

vorliegt.

9. Verfahren zur Herstellung einer Verbindung der Formel (I) gemäss Anspruch 1, dadurch gekennzeichnet, dass man ein Allen-Carbinol der Formel

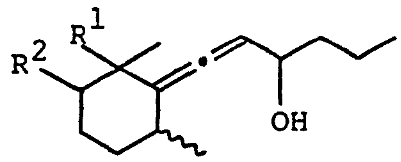

(II)

in welcher ~ eine cis oder trans Kohlenstoff-Kohlenstoff Einfachbindung darstellt, mittels einer katalytischen Hydrierung in homogener oder heterogener Phase bei einem Druck, der höher als atmosphärischer Druck ist, in Anwesenheit eines Edelmetalls reduziert.

10. Verfahren gemäss Anspruch 9, dadurch gekennzeichnet, dass der Katalysator Palladium oder Platin auf Kohle ist.

11. Verfahren zur Herstellung einer Verbindung der Formel (I) gemäss Anspruch 1 hauptsächlich als Trans-Isomer der Formel

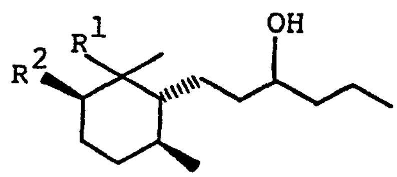

dadurch gekennzeichnet, dass man ein Keton der Formel

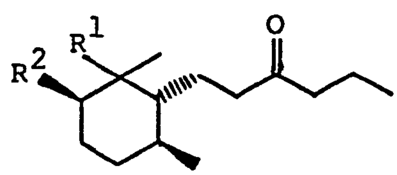

(III)

mittels eines Alkalimetall-aluminohydrids reduziert.

**Claims**

1. Compound of formula

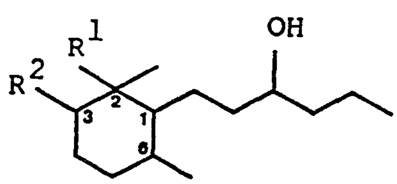

(I)

wherein each of symbols $R^1$ and $R^2$ designates a methyl radical or wherein $R^1$ designates an ethyl radical and $R^2$ a methyl radical or hydrogen.

2. 1-(2,2,3,6-Tetramethyl-1-cyclohexyl)-3-hexanol.

3. 1-(2-Ethyl-2,6-dimethyl-1-cyclohexyl)-3-hexanol.

4. 1-(2-Ethyl-2,3,6-trimethyl-1-cyclohexyl)-3-hexanol.

5. Compound according to claim 2 of formula

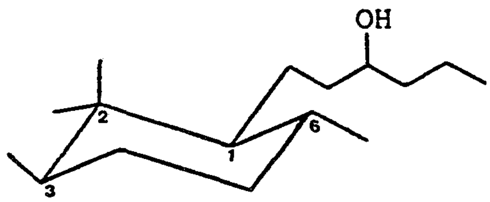

6. Compound according to claim 2 of formula

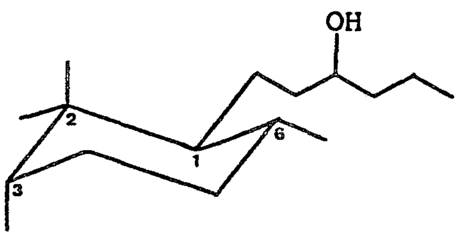

7. Utilization of a compound of formula (I) according to claim 1 as perfuming ingredient.

8. Utilization according to claim 7, characterized in that the compound of formula (I) is essentially in its trans isomeric form defined by the following formulas:

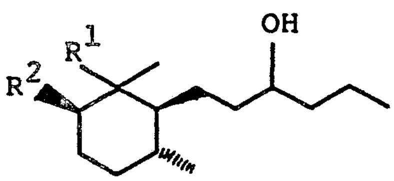 (a)

and

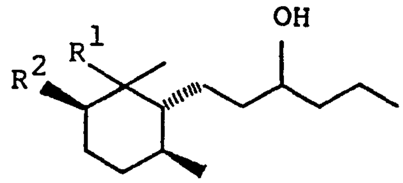 (d)

9. Process for the preparation of a compound of formula (I) according to claim 1, characterized in that the allenic carbinol of formula

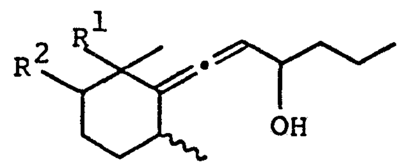 (II)

wherein ~ stands for a cis or trans signel carbon-carbon bond is reduced by catalytic hydrogenation in homogeneous or heterogeneous phase, at a pressure higher than the atmospheric pressure and in the presence of a noble metal catalyst.

10. Process according to claim 9, characterized in that the catalyst is palladium or platinum on charcoal.

11. Process for the preparation of a compound of formula (I) according to claim 1 essentially in its trans isomeric form of formula

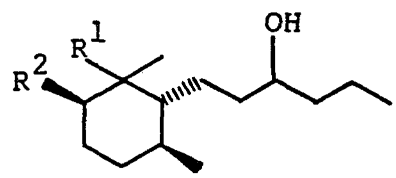

characterized in that the ketone of formula

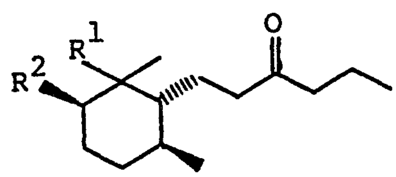 (III)

is reduced by means of an alkali metal aluminohydride.